# DEMANDE DE BREVET EUROPEEN

(11) **EP 1 068 873 A1**
(43) Date de publication de la demande: **17.01.2001**
(21) Numéro de dépôt: 00401971.7
(22) Date de dépôt: 07.07.2000
(51) Int. Cl.: A61M 1/16, C11D 1/72, C11D 3/39, A01N 37/16

(54) **Nouveau procédé de nettoyage et désinfection d'un appareil d'hémodialyse avec une solution aqueuse comprenant un agent tensioactif et un générateur d'oxygène actif et nouvelle composition**

(30) Priorité: 13.07.1999 FR 9909102
(71) Demandeur: SOCIETE D'EXPLOITATION DE PRODUITS POUR LES INDUSTRIES CHIMIQUES, S.E.P.P.I.C., 75321 Paris Cédex 07 (FR); Bioxal, 75007 Paris (FR)
(72) Inventeur: Gouges, Yves, 75014 Paris (FR); Gamet, Jean-Claude, 18400 Saint Florent sur Cher (FR); Le Rouzic, Daniel, 95120 Ermont (FR); Ascarateil, Stéphane, 95150 Taverny (FR); Steinhauer, Udo, 92190 Meudon (FR)
(74) Mandataire: Conan, Philippe Claude

(57) **Abrégé**

Utilisation d'une solution aqueuse comprenant un agent tensioactif, de formule (I) :

R₁-O-[CH(R₂)-CH(R₃)-O]ₙ-R₄ (I)

dans laquelle R₁ représente un radical hydrocarboné linéaire ou ramifié, saturé ou insaturé comprenant de 5 à 31 atomes de carbones, R₂ et R₃ représentent indépendamment l'un de l'autre, un atome d'hydrogène ou un radical alkyle comprenant 1 ou 2 atomes de carbone, R₄ représente un atome d'hydrogène, un radical alkyle linéaire ou ramifié comprenant de 1 à 4 atomes de carbone ou un radical benzyle, et n représente un nombre entier compris entre 1 et 50 et de préférence inférieur à 20, ou un mélange desdits composés de formule (I), pour nettoyer un, plusieurs ou tous les éléments de la chaîne de dialyse d'un appareil d'hémodialyse. Procédé mettant en oeuvre ledit agent tensioactif, ainsi qu'un agent générateur d'oxygène actif ; utilisation d'une solution aqueuse comprenant ledit agent tensioactif et ledit agent générateur d'oxygène actif ; nouvelle solution aqueuse les contenant.

## Description

L'invention a pour objet, un nouveau procédé de désinfection des appareils d'hémodialyse.

Le principe général de la dialyse extra corporelle repose sur des échanges à travers une membrane semi-perméable, entre le sang à épurer et un dialysât de composition électrolytique proche du liquide extracellulaire normal, qui est donc constitué d'eau et d'électrolytes en concentrations suffisantes pour que la concentration en électrolytes dans le sang, en fin de dialyse soit normale. Pour que des échanges efficaces puissent se faire, il faut en permanence, renouveler le sang et le dialysât, car la vitesse de diffusion ou de dialyse, est fonction de la différence de concentrations entre ces deux liquides.

Tout appareil de dialyse est constitué d'un générateur, d'un dialyseur, ou rein artificiel, et d'un circuit d'eau. Cet ensemble d'éléments sera appelé, dans l'exposé suivant, chaîne de dialyse.

Le générateur a pour fonctions, d'assurer la préparation d'un dialysât réchauffé à 37°C en mélangeant, l'eau, une solution d'électrolytes, dont la concentration ne permet pas la prolifération d'agents infectieux, et la solution tampon, de faire circuler le sang et le dialysât et de permettre de contrôler les paramètres de dialyse.

L'eau utilisée dans ces appareils devant être très pure et conforme à la Pharmacopée européenne, elle subit un traitement qui comporte une succession d'étapes, comme la filtration, le passage sur résines, le passage sur charbon actif, l'osmose ou la déionisation.

Il existe différents types de générateurs. Les générateurs d'hémodialyse utilisant la technique du dialysât en circuit ouvert, permettent d'obtenir un dialysât de meilleure qualité bactériologique, que ceux utilisant la technique du circuit fermé, dans laquelle il y a recirculation du dialysât.

Le dialyseur a pour fonction d'éliminer l'eau et les déchets organiques provenant du sang du corps humain et migrant du sang vers le dialysât à travers la membrane semi-perméable. Cette membrane semi-perméable peut être disposée en plaques, en compartiments empilés les uns sur les autres, en forme de bobines, en compartiment unique enroulé en spirale autour d'un axe ou en ensembles de capillaires ; cette membrane peut être organique en cellophane, cuprophane, polysulfone, polypropylène, polyamide, polyacétate, ou en copolymère d'acrylonitrile. Le dialyseur est livré stérile et est souvent à usage unique, comme en France ; cependant dans certains pays le dialyseur est réutilisé. Comme types de dialyseurs, on peut citer les reins bobines, comprenant un enroulement parallèle de deux feuillets de membranes dialysantes en spirale autour d'un axe, le sang circulant à l'intérieur de l'enveloppe formée par les deux feuillets de la membrane et le dialysât à l'extérieur ; les reins à fibres creuses opposant une résistance très faible au passage du sang ; ou les reins plaques comprenant 15 à 20 feuillets assemblés en plaques.

L'utilisation de solutions aqueuses d'acide peracétique pour désinfecter tous les éléments de la chaîne de dialyse a été décrite depuis de nombreuses années. On peut citer par exemple L.J. Fischbach, AAMI Technol. Anal Rev. 1985, 15-18 ; Von Sprößig et al. Dtsch. Ges.wesen 27 (1972), H 23 1085-1089 ; R. Nicolle et al, EP 0370850, publié le 30 mai 1991).

Cependant, le souhait de la clientèle, tant hospitalière, qu'associative ou privée, de procéder à des séances d'hémodialyse, à la fois plus courtes et plus efficaces, a conduit les constructeurs de machines à mettre en oeuvre des membranes à haute perméabilité et à mettre en application dans des appareils à hauts flux, des techniques d'échanges à forte convection, comme l'ultrafiltration, ce qui engendre l'apparition de dépôts protéiques et lipidiques issus du sang des patients, dans les circuits des générateurs de dialysât. A la longue, ses dépôts obstruent les réseaux d'évacuation d'eau et bloquent le bon fonctionnement de la machine de dialyse, voire du service tout entier. Or, si les solutions aqueuses d'acide peracétique possèdent de bonnes propriétés désinfectantes, elles ne sont pas suffisamment détergentes pour éliminer de tels dépôts, et ceci malgré la capacité du peroxyde d'hydrogène à dénaturer certaines protéines. Pour cette raison, les appareils sont aujourd'hui encore nettoyés périodiquement avec des solutions basiques, telles que l'eau de Javel, ce qui n'est pas satisfaisant, sous l'angle toxicologique et environnemental.

C'est pourquoi, la demanderesse a cherché à mettre au point un procédé intégrant à la fois la désinfection, le détartrage et le nettoyage des appareils de dialyse, qui permette de limiter la fréquence des traitements alcalins intermédiaires, voire de les éliminer. Elle a découvert de manière inattendue et contrairement à un préjugé largement répandu, que l'on pouvait utiliser un agent tensioactif, pour nettoyer la chaîne de dialyse des appareils d'hémodialyse, sans rencontrer de problèmes de corrosion, sans générer de mousse, ce qui rend difficile à l'élimination du produit nettoyant par rinçage, et ceci, malgré la température de nettoyage, généralement autour de 37°C et en toute sécurité pour les patients.

Selon un premier aspect, l'invention a donc pour objet, l'utilisation d'une solution aqueuse comprenant un agent tensioactif, pour nettoyer un, plusieurs ou tous les éléments de la chaîne de dialyse d'un appareil d'hémodialyse.

Le phénomène de détergence généralement impliqué dans le nettoyage de surfaces dures, comprend quatre étapes successives : une étape de mouillage de la souillure et de son support, une étape de rupture de la liaison support-souillure, une phase de dispersion de cette souillure et le maintien de cette souillure en suspension.

Ce phénomène procède de nombreuses propriétés physico-chimiques des agents tensioactifs, parmi lesquelles on peut citer, leur pouvoir mouillant, leur pouvoir émulsionnant et leur pouvoir dispersant. Il n'est cependant pas déductible d'une ou de l'autre ou d'une combinaison de ces propriétés.

Dans la présente description, le terme, un agent tensioactif, s'entend comme désignant ou bien un seul composé chimique ou bien un mélange de composés chimiques.

Selon les dispositions réglementaires en vigueur dans le pays, dans lequel est effectué le nettoyage de l'appareil, et notamment selon la possibilité ou l'impossibilité qu'il y a, de réutiliser le dialyseur, cette opération va inclure ou exclure cet élément de la chaîne de dialyse.

Dans le cadre de la présente invention, les agents tensioactifs préférés sont les agents tensioactifs non ioniques.

L'agent tensioactif non ionique est notamment choisi, parmi ceux décrits dans la demande de brevet européen publiée sous le numéro WO 98/37762, et plus particulièrement choisis parmi les produits commerciaux suivants : le GENAPOL™ 2822, le GENAPOL™ 2908, le GENAPOL™ 2908D, le GENAPOL™ 2909, le TRITON™ DF12, le TRITON™ DF16, le TRITON™ CF10, le DOWFAX™ 20B102, l'AKYPO™ RO 90, le SYNPERONIC™ LF RA 30 ou le SIMULSOL™ NW 900, l'AKYPO LF2 ou l'AKYPO LF4. Ces produits, disponibles dans le commerce, ont la composition chimique suivante :

| **Nom commercial** | **Composition chimique** |
|---|---|
| GENAPOL™ 2822 | Mélange d'alcools alkoxylés en C₁₀, C₁₂, C₁₄ (5OE, 4OP) |
| GENAPOL™ 2908D | Mélange d'alcools alkoxylés en C₁₁, C₁₃, C₁₅ (6 à 7OE, 3OP) |
| GENAPOL™ 2909 | Mélange d'alcools alkoxylés en C₁₂, C₁₄ (5OE, 3OP) |
| TRITON™ DF12 | Ethers benzyliques d'alcools alkoxylés en C₈, C₁₀ (2OE, 5OP) |
| TRITON™ DF16 | Mélange d'alcools alkoxylés en C₈, C₁₀ (6OE, 3OP) |
| TRITON™ CF10 | Ether benzylique d'alcools éthoxylés en C₈ (16OE) |
| AKYPO™ RO 90 | Mélange d'éthers carboxyliques éthoxylés en C₁₆ C₁₈ (9 OE) |
| DOWFAX™ 20B102 | |
| SIMULSOL™ NW 900 | Mélange d'alcools alkoxylés (x OE, y OP) |
| AKYPO LF2 | Ether carboxylique éthoxylé en C₈ (8 OE) |
| AKYPO LF4 | Mélange d'éthers carboxyliques éthoxylés en C₆, C₈ (7 OE) |

Dans le cadre de la présente invention, les agents tensioactifs non ioniques préférés sont ceux de formule (I) suivante :

R₁-O-[CH(R₂)-CH(R₃)-O]ₙ-R₄ (I)

dans laquelle R₁ représente un radical hydrocarboné linéaire ou ramifié, saturé ou insaturé comprenant de 5 à 31 atomes de carbones, R₂ et R₃ représentent indépendamment l'un de l'autre, un atome d'hydrogène ou un radical alkyle comprenant 1 ou 2 atomes de carbone, R₄ représente un atome d'hydrogène, un radical alkyle linéaire ou ramifié comprenant de 1 à 4 atomes de carbone ou un radical benzyle, et n représente un nombre entier compris entre 1 et 50 et de préférence inférieur à 20.

Par radical hydrocarboné linéaire ou ramifié, saturé ou insaturé et comprenant de 5 à 31 atomes de carbone, on désigne pour R₁, dans la formule (I) telle que définie précédemment, notamment les radicaux alkyles ou les radicaux alkènyles.

R₁ représente plus particulièrement un radical choisi parmi les radicaux hexyle, heptyle, octyle, nonyle, décyle, undécyle, undécènyle, dodécyle, tridécyle, tetradécyle, pentadécyle, hexadécyle, heptadécyle, octadécyle, octadécènyle, octadécatriènyle, octadécadiènyle, eicosanyle ou béhènyle, lesdits radicaux étant linéaires ou ramifiés.

Selon un aspect particulier de la présente invention, R₁ représente un radical choisi de préférence parmi les radicaux alkyles linéaires ou ramifiés comportant de 8 à 18 atomes de carbone.

Par radical alkyle comprenant de 1 à 4 atomes de carbone, on désigne pour R₄, dans la formule (I) telle que définie précédemment, les radicaux méthyle, éthyle, propyle ou butyle, linéaires ou ramifiés.

Le groupe divalent radical -[CH(R₂)-CH(R₃)-O]ₙ- représente, soit une chaîne composée uniquement de groupes éthoxyle (R₂ et R₃ = H), soit une chaîne composée uniquement de groupes propoxyle (R₂ ou R₃ = CH₃), soit une chaîne composée uniquement de groupes butoxyle (R₂ et R₃ = CH₃) soit une chaîne composée d'un mélange de deux sortes ou des trois sortes de groupes énoncés ci-dessus. Dans ces derniers cas, les fragments -CH₂-CH₂-O-, -CH(CH₃)-CH₂-O-et -CH(CH₃)-CH(CH₃)-O-, sont distribués dans ladite chaîne, de façon séquencée ou aléatoire. Dans la présente description, le nombre de groupes éthoxyle par molécule est appelé indice d'OE et le nombre de groupes propoxyle par molécule est appelé indice d'OP.

Dans le cadre de la présente invention l'un des agents tensioactifs préférés, est un mélange d'alcools gras comportant 11, 13 et 15 atomes de carbone, éthoxylés et propoxylés, avec un indice d'OE environ compris entre 6 et 7 et un indice d'OP environ égal à 3.

La concentration efficace de l'agent tensioactif dans la solution aqueuse nettoyante, dépend essentiellement des propriétés physico-chimiques de cet agent.

Lors du cycle de nettoyage à l'intérieur de la chaîne de dialyse, le composé de formule (I) ou le mélange de composés de formule (I), est à une concentration généralement, comprise entre 20 et 100 ppm (environ entre 0,002% en poids et 0,01% en poids) de préférence entre 30 et 50 ppm (environ entre 0,003% en poids et 0,005% en poids).

La solution mise oeuvre dans le procédé selon l'invention est généralement préparée par dilution dans le générateur, de solutions plus concentrées disponibles commercialement ou aisément préparées par l'homme du métier. Les appareils procèdent à des dilutions automatiques des solutions comprises environ entre le 1/10ème et le 1/50ème et plus particulièrement entre le 1/30ème et le 1/40ème.

Selon la machine utilisée, il est parfois nécessaire de diluer préalablement la solution initiale avant de l'y introduire, de manière à ce que la dilution par la machine dans l'intervalle de taux indiqué ci-dessus, conduise à une solution d'agent tensioactif à une concentration telle que définie précédemment.

La mise en oeuvre de cette application dépend des caractéristiques techniques de l'appareil à nettoyer. La solution aqueuse est généralement aspirée par la machine, à travers une canne d'aspiration plongée dans le récipient la contenant. Le cycle de nettoyage dure de 10 à 30 minutes ; il consiste en une circulation de la solution préalablement diluée par le générateur, dans tout le circuit dans lequel circule du dialysât, lors d'une séance de dialyse ; à la suite de ce cycle de nettoyage, on procède à un cycle de désinfection.

Selon un autre aspect de la présente invention, celle-ci a pour objet un procédé de nettoyage, de détartrage et de désinfection d'un ou plusieurs éléments d'une chaîne de dialyse, comprenant au moins une séquence de traitement consistant en, une étape de nettoyage, avec une solution aqueuse d'un agent tensioactif telle que définie précédemment et une étape de désinfection avec une solution aqueuse d'un agent désinfectant générateur d'oxygène actif.

Selon un aspect préféré du procédé tel que défini ci-dessus l'étape de nettoyage précède l'étape de détartrage et de désinfection.

Par au moins une séquence, on indique que ladite séquence peut être mise en oeuvre une ou plusieurs fois consécutivement, selon l'état de saleté de ladite chaîne de dialyse.

Par agent désinfectant générateur d'oxygène actif, on désigne notamment les peroxydes et plus particulièrement le peroxyde d'hydrogène seul ou en présence d'un ou plusieurs acides peroxycarboxyliques. Comme acide peroxycarboxylique, on désigne plus particulièrement, les acides paralcanoïques comportant de 1 à 6 atomes de carbone, et tout particulièrement, les acides performique, peracétique, perpropionique ou perbutyrique.

Selon une variante préférée du procédé tel que défini précédemment, la solution aqueuse d'agent désinfectant mise en oeuvre, est une solution comprenant de l'acide peracétique, du peroxyde d'hydrogène et de l'acide acétique.

La quantité efficace d'agent désinfectant dépend de sa composition chimique. Lors de la séquence de nettoyage et de désinfection de la chaîne de dialyse, lorsqu'il s'agit d'une solution comprenant de l'acide peracétique, du peroxyde d'hydrogène et de l'acide acétique, l'acide peracétique est à une concentration généralement comprise entre environ 25 ppm et 500 ppm (entre environ 0,0025% en poids et environ 0,05% en poids) et, de préférence, entre 100 ppm et 250 ppm (entre environ 0,01% en poids et environ 0,025% en poids) ; le peroxyde d'hydrogène est à une concentration généralement comprise entre, environ 250 ppm et 5000 ppm (entre environ 0,025% en poids et environ 0,5% en poids ) et, de préférence entre 1000 ppm et 2500 ppm (entre environ 0,1% en poids et environ 0,25% en poids). ; l'acide acétique est à une concentration généralement comprise entre, environ, 500 ppm et 5000 ppm (entre environ 0,05% en poids et environ 0,5% en poids ) et de préférence entre 1000 ppm et 2500 ppm (entre environ 0,1% en poids et environ 0,25% en poids).

La solution mise oeuvre dans le procédé selon l'invention est généralement préparée par dilution dans le générateur, de solutions plus concentrées disponibles commercialement ou aisément préparées par l'homme du métier. Les appareils procèdent à des dilutions automatiques des solutions, comprises environ entre le 1/10ème et le 1/50ème et plus particulièrement entre le 1/30ème et le 1/40ème.

Selon la machine utilisée, il est parfois nécessaire de diluer préalablement la solution initiale avant de l'y introduire. Dans le cadre de la présente invention, on préfère utiliser des solutions prêtes à l'emploi, qui ne nécessitent pas de dilution préalable à leur introduction dans l'appareil d'hémodialyse.

Le procédé est généralement mis en oeuvre à une température comprise entre 30°C et 50°C et plus particulièrement à environ 37°C.

Comme solution d'agent désinfectant prête à être diluée par l'appareil, il y a par exemple, les solutions aqueuses prêtes à l'emploi comprenant de 0,1% à 1% en poids, d'acide peracétique, de 2% à 10% en poids de peroxyde d'hydrogène et de 2 à 10% en poids d'acide acétique, comme les solutions aqueuses prêtes à l'emploi comprenant de 0,1% à 1% en poids, d'acide peracétique, de 6% à 8% en poids de peroxyde d'hydrogène et de 2 à 10% en poids d'acide acétique, décrites dans le brevet français publié sous le numéro 2 639 233. Parmi celles-ci, il y a plus particulièrement les solutions aqueuses comprenant 0,1% à 0,6% en poids, d'acide peracétique, de 3% à 7% en poids de peroxyde d'hydrogène et de 3 à 6% en poids d'acide acétique. Comme solutions aqueuses commerciales prêtes à l'emploi, il y a par exemple, celle comprenant environ 0,35% en poids, d'acide peracétique, environ 7% en poids de peroxyde d'hydrogène et environ 3,5% en poids d'acide, commercialisée sous le nom DIALOX™ ou la solution aqueuse comprenant environ 0,5% en poids d'acide peracétique, environ 10% en poids de peroxyde d'hydrogène et environ 6% en poids d'acide acétique, commercialisée sous le nom OXAGAL™ ou encore la solution aqueuse comportant en autre environ 0,9% d'acide peracétique et 9% de peroxyde d'hydrogène, commercialisée sous le nom de DOXAN™.

La solution désinfectante mise en oeuvre dans le procédé tel que défini précédemment, comprend éventuellement, un ou plusieurs additifs connus de l'homme du métier et à des concentrations usuelles, choisis par exemple parmi les agents stabilisants, les acides forts, les agents séquestrant, les inhibiteurs de corrosion ou les capteurs de radicaux libres.

C'est pourquoi l'invention a aussi pour objet, l'utilisation d'une solution aqueuse comprenant de 0,1% à 1% en poids, d'acide peracétique, de 2% à 10% en poids de peroxyde d'hydrogène et de 2 à 10% en poids d'acide acétique, pour, après une dilution dans l'appareil, comprise entre le 1/10ème et le 1/50ème et plus particulièrement comprise entre le 1/30ème et le 1/40ème, détartrer et désinfecter un, plusieurs ou tous les éléments de la chaîne de dialyse d'un appareil d'hémodialyse selon le procédé tel que défini précédemment.

Selon une variante préférée du procédé tel que défini ci-dessus, l'étape de nettoyage et l'étape de détartrage et de désinfection sont simultanées.

Selon cette dernière variante, la séquence telle que définie ci-dessus ne comporte qu'une seule étape, au cours de laquelle la chaîne de dialyse est nettoyée désinfectée et détartrée, par la mise en oeuvre d'une solution aqueuse comprenant à la fois, un agent désinfectant générateur d'oxygène actif et un agent tensioactif, tels qu'ils ont été définis précédemment.

C'est pourquoi l'invention a aussi pour objet, l'utilisation d'une solution aqueuse comprenant un agent désinfectant générateur d'oxygène actif tel que défini précédemment et un agent tensioactif tel que défini précédemment pour nettoyer désinfecter et détartrer un, plusieurs ou tous les éléments de la chaîne de dialyse.

De telles solutions sont préparées par des méthodes connues de l'homme du métier comme, par exemple, par addition de l'agent tensioactif dans la solution contenant le générateur d'oxygène actif.

Comme utilisation préférée, on préfère celle d'une solution aqueuse comprenant de 0,1% à 1% en poids, d'acide peracétique, de 2% à 10% en poids de peroxyde d'hydrogène et de 2 à 10% en poids d'acide acétique et de 0,02% en poids à 0,5% en poids, d'un composé de formule (I) ou d'un mélange de composés de formule (I) telle que définie précédemment, pour, après une dilution comprise entre le 1/10ème et le 1/50ème et plus particulièrement comprise entre le 1/30ème et le 1/40ème, nettoyer, détartrer et désinfecter un, plusieurs ou tous les éléments de la chaîne de dialyse d'un appareil d'hémodialyse, selon le procédé tel que défini précédemment et, plus particulièrement, l'utilisation telle que définie ci-dessus, d'une solution aqueuse comprenant de 0,1% à 0,6% en poids, d'acide peracétique, de 3% à 7% en poids de peroxyde d'hydrogène et de 3% à 6% en poids d'acide acétique et de 0,05% en poids à 0,15% en poids, d'un agent tensioactif consistant essentiellement en un mélange d'alcools gras comportant 11, 13 et 15 atomes de carbone, éthoxylés et propoxylés, avec un indice d'OE environ compris entre 6 et 7 et un indice d'OP environ égal à 3. Une telle solution, qui comprend éventuellement un ou plusieurs additifs connus de l'homme du métier, tels que les agents stabilisants, les acides forts, les agents séquestrant, les inhibiteurs de corrosion ou les capteurs de radicaux libres, fait aussi partie intégrante de la présente invention.

De telles solutions sont préparées par des méthodes connues de l'homme du métier comme, par exemple, par addition de l'agent tensioactif dans une solution aqueuse comprenant l'acide peracétique, l'acide acétique le peroxyde d'hydrogène et éventuellement un ou plusieurs additifs connus de l'homme du métier, choisis parmi les agents stabilisants, les acides forts, les agents séquestrant, les inhibiteurs de corrosion ou les capteurs de radicaux libres.

La mise en oeuvre de cette application dépend notamment des caractéristiques techniques de l'appareil à nettoyer. La solution aqueuse contenant l'agent désinfectant et l'agent tensioactif est en général aspirée par la machine, à travers une canne d'aspiration plongée dans le récipient la contenant. Le cycle de nettoyage, détartrage et désinfection dure de 10 à 30 minutes à des dilutions au 1/35ème ; il consiste en une circulation de la solution préalablement diluée par le générateur, dans tout le circuit dans lequel circule le dialysât lors d'une séance de dialyse. Au Japon, la mise en oeuvre de cette application comporte un temps contact d'une nuit et la solution utilisée est diluée au 1/40ème voir au 1/50ème par la machine.

Le procédé objet de la présente invention et ses variantes comprend généralement aussi une étape de rinçage

Le rinçage avec de l'eau ultra pure a pour objectif d'éliminer les traces de d'agent tensioactif et/ou d'agent désinfectant. Le cycle de rinçage varie selon les machines de 10 à 50 minutes.

Selon un autre objet de la présente invention celle-ci a pour objet un procédé pour conserver la chaîne de dialyse à l'abri du développement du bio-film pendant une période de temps allant jusqu'à plusieurs jours, caractérisé en ce que ladite chaîne est remplie avec une solution nettoyante et désinfectante telle que définie précédemment et laissée au repos sans recirculation de ladite solution.

Les exemples suivants illustrent l'invention sans toutefois la limiter.

### Exemple 1

Une solution A comprenant 0,1% en poids de GENAPOL™ 2908D, entre 0,3 et 0,4% en poids d'acide peracétique, entre 6.5% et 7% en poids de peroxyde d'hydrogène et entre 3% et 4% en poids d'acide acétique a été utilisée pendant une période de 6 semaines, comme agent désinfectant détartrant et nettoyant dans un centre de dialyse et ses effets ont été comparés avec ceux d'une solution B comprenant 0,9% d'acide peracétique et 9% de peroxyde d'hydrogène.

La technique utilisée par ce centre, était l'hémodialyse conventionnelle avec des membranes HF. Les machines de ce centre ne sont pas équipées de pré-filtres destinés à fabriquer du dialysât apyrogène au moment de son utilisation, les méthodes d'ultrafiltration ou de diafiltration n'y sont donc pas appliquées.

### Essai préliminaire

Comme essai préliminaire, on a mesuré la tension superficielle de la solution A concentrée, de la solution A diluée au 1/35ème, lors d'un cycle de nettoyage et désinfection (appelé dans le tableau suivant, traitement) et de l'eau circulant à l'issue d'un cycle de rinçage dans différents dialyseurs. On a obtenu les résultats suivants :

| DIALYSEUR | solution A concentrée | solution diluée (pendant la désinfection) | solution (à l'issue du rinçage) |
|---|---|---|---|
| ALTHIN™ Série 1000 après 15mn de traitement temps de rinçage : 7 mn | 33,0 mN/m | 33,1 mN/m | 71,4mN/m |
| FRESENIUS™ 2008 après 9mn de traitement temps de rinçage : 20 mn | 33,5 mN/m | 32,9 mN/m | 72,2mN/m |

Cet essai montre, qu'à l'issue du cycle de rinçage opéré par la machine, la tension superficielle de l'eau y circulant, est pratiquement identique à celle mesurée d'un échantillon d'eau ultra-purifiée par osmose inverse (environ 72 mN/m). Ceci démontre que la solution A est entièrement évacuée de l'appareil après le rinçage.

### Essai principal

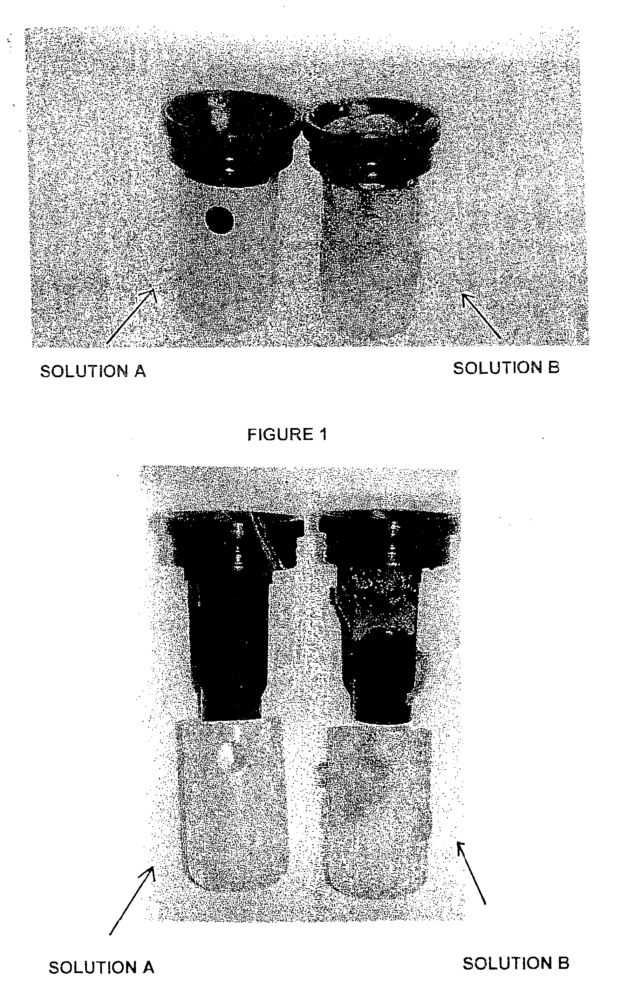

On a comparé sur deux machines identiques et dans des conditions opératoires identiques, le pouvoir nettoyant de la solution A avec celui de la solution B sur une période de 6 semaines, sans qu'aucun nettoyage intermédiaire à l'eau de javel ne soit effectué dans le cas de la solution A et avec un ou deux nettoyages par semaine avec la solution B. Les photos du siphon se trouvant en sortie du conduit d'évacuation de deux dialyseurs font apparaître clairement la supériorité de l'activité nettoyante de la solution A seule, par rapport à celle de la solution B. combinée avec des séances de nettoyage à l'eau de Javel (Figures 1 et 2). Cette comparaison a été effectuée avec 2 machines de type FRESENIUS 2008 C et 2 machines de type ALTHIN ™ SERIE 1000.

### Exemple 2

Des essais complémentaires ont été réalisés, avec pour but d'effectuer des cycles de désinfection identiques à ceux rencontrés dans les conditions d'utilisation des produits désinfectants, et de suivre le rinçage de la solution A sur des appareils de type Gambro™ avec préfiltres. Les phases retenues simulaient la désinfection entre deux patients, le rinçage (ces deux phases étant programmées automatiquement par la machine), la purge du dialyseur par le dialysât (d'une durée de 20 minutes et imposée pour éviter l'introduction de bulles d'air dans le sang du patient) et une stagnation de nuit. Les essais ont porté sur deux jours et six cycles de désinfection au total.

A l'issue de ces essais, il est apparu que la tension superficielle de la solution était quasiment identique à celle de l'eau ultra-pure de comparaison et que le compartiment sang du dialyseur, donc le patient, n'avait jamais été jamais en contact avec la solution A avec ce type d'appareil.

### MATERIELS et METHODES

Le générateur choisi est l'AK 200 ULTRA de Gambro™. Il est équipé de deux pré-filtres Ultrafiltre™ U 8000 S, dont la membrane est essentiellement composée de polyaryléther. Ses caractéristiques de fonctionnement sont les suivantes :
- Volume aspiré en mode on line : 120ml ( désinfection chimique - peracétique)
- Temps de contact dans la machine : 10 minutes à la température de 30°C
- Temps de rinçage en mode on line : 45 minutes ;
- Dilution : 1/35ème

Le rinçage n'est pas continu mais composé de 4 phases, dont les trois dernières sont réalisées à la température de 80°C.

Le dialyseur utilisé est le Polyfiltre™11S composé de polyamides. Ce filtre n'a été changé que le dernier test, ceci pour se placer dans les pires conditions possibles au regard de l'accumulation de l'agent tensioactif. Dans les conditions normales d'utilisation le dialyseur est à usage unique ( en Europe ).

Des prélèvements du milieu circulant ont été effectués durant les phases de rinçage du générateur et de purge du dialyseur au niveau du dispositif d'injection ou à la sortie égout de la machine. Un rinçage final du dialyseur a été effectué afin de récupérer les traces éventuelles de tensioactif à l'intérieur du compartiment sang. On a déterminé la tension superficielle des divers prélèvements.

## Revendications

1. Utilisation d'une solution aqueuse comprenant un agent tensioactif, pour nettoyer un, plusieurs ou tous les éléments de la chaîne de dialyse d'un appareil d'hémodialyse.

2. Utilisation telle que définie à la revendication 1, pour laquelle l'agent tensioactif est un agent tensioactif non ionique.

3. Utilisation telle que définie à la revendication 2, pour laquelle l'agent tensioactif non ionique est un composé de formule (I) suivante :
R₁-O-[CH(R₂)-CH(R₃)-O]ₙ-R₄ (I)
dans laquelle R₁ représente un radical hydrocarboné linéaire ou ramifié, saturé ou insaturé comprenant de 5 à 31 atomes de carbones, R₂ et R₃ représentent indépendamment l'un de l'autre, un atome d'hydrogène ou un radical alkyle comprenant 1 ou 2 atomes de carbone, R₄ représente un atome d'hydrogène, un radical alkyle linéaire ou ramifié comprenant de 1 à 4 atomes de carbone ou un radical benzyle, et n représente un nombre entier compris entre 1 et 50 et de préférence inférieur à 20, ou un mélange desdits composés de formule (I).

4. Utilisation telle que définie à la revendication 3, pour laquelle, dans la formule (I), R₁ représente un radical choisi de préférence parmi les radicaux alkyles linéaires ou ramifiés comportant de 8 à 18 atomes de carbone.

5. Utilisation telle que définie à la revendication 4, pour laquelle, l'agent tensioactif, est un mélange d'alcools gras comportant 11, 13 et 15 atomes de carbone, éthoxylés et propoxylés, avec un indice d'OE environ compris entre 6 et 7 et un indice d'OP environ égal à 3.

6. Utilisation telle que définie à l'une des revendications 3 à 5, d'une solution aqueuse comprenant entre environ 0,002% en poids et environ 0,01% en poids, de préférence entre environ 0,003% en poids et 0,005% en poids d'un composé de formule(I) ou d'un mélange de composés de formule (I).

7. Procédé de nettoyage, de détartrage et de désinfection d'un, de plusieurs ou de tous les éléments d'une chaîne de dialyse, comprenant au moins une séquence de traitement consistant en une étape de nettoyage avec une solution aqueuse d'un agent tensioactif, telle que définie à l'une des revendications 2 à 5, et une étape de détartrage et de désinfection avec une solution aqueuse d'un agent générateur d'oxygène actif

8. Procédé tel que défini à la revendication 7, dans lequel l'étape de nettoyage précède l'étape de détartrage et de désinfection.

9. Procédé tel que défini à l'une des revendications 7 ou 8, dans lequel la solution aqueuse mise en oeuvre dans l'étape de détartrage et de désinfection, est une solution comprenant de l'acide peracétique, du peroxyde d'hydrogène et de l'acide acétique.

10. Procédé tel que défini à la revendication 9, pour lequel, dans la solution aqueuse mise en oeuvre dans l'étape de détartrage et de désinfection, l'acide peracétique est à une concentration comprise entre environ 0,0025% en poids et environ 0,05% en poids, le peroxyde d'hydrogène est à une concentration comprise entre environ 0,025% en poids et environ 0,5% en poids et l'acide acétique est à une concentration comprise entre environ 0,05% en poids et environ 0,5% en poids.

11. Procédé tel que défini à la revendication 10, pour lequel, dans la solution aqueuse mise en oeuvre dans l'étape de détartrage et de désinfection, l'acide peracétique est à une concentration comprise entre environ 0,01% en poids et environ 0,025% en poids, le peroxyde d'hydrogène est à une concentration comprise entre environ 0,1% en poids et environ 0,25% en poids et l'acide acétique est à une concentration comprise entre environ 0,1% en poids et environ 0,25% en poids.

12. Procédé tel que défini à l'une des revendications 7 à 11 dans lequel dans lequel l'étape de tartrage et de désinfection est mise en oeuvre avec une solution aqueuse comprenant de 0,1% à 1% en poids, d'acide peracétique, de 2% à 10% en poids de peroxyde d'hydrogène et de 2 à 10% en poids d'acide acétique, que l'on introduit dans l'appareil d'hémodialyse, qui est diluée dans ledit appareil entre le 1/10ème et le 1/50ème et plus particulièrement comprise entre le 1/30ème et le 1/40ème, puis mise à circuler dans un, plusieurs ou tous les éléments de la chaîne de dialyse.

13. Procédé tel que défini à la revendication 7, pour lequel, dans la séquence de traitement, l'étape de nettoyage et l'étape de détartrage et désinfection sont simultanées.

14. Utilisation d'une solution aqueuse comprenant un agent générateur d'oxygène actif et un agent tensioactif, pour nettoyer, désinfecter et détartrer un, plusieurs ou tous les éléments de la chaîne de dialyse.

15. Utilisation telle que définie à la revendication 16, pour laquelle l'agent tensioactif est un agent tensioactif non ionique.

16. Utilisation telle que définie à la revendication 15, pour laquelle l'agent tensioactif non ionique est un composé de formule (I) suivante :
R₁-O-[CH(R₂)-CH(R₃)-O]ₙ-R₄ (I)
dans laquelle R₁ représente un radical hydrocarboné linéaire ou ramifié, saturé ou insaturé comprenant de 5 à 31 atomes de carbones, R₂ et R₃ représentent indépendamment l'un de l'autre, un atome d'hydrogène ou un radical alkyle comprenant 1 ou 2 atomes de carbone, R₄ représente un atome d'hydrogène, un radical alkyle linéaire ou ramifié comprenant de 1 à 4 atomes de carbone ou un radical benzyle, et n représente un nombre entier compris entre 1 et 50 et de préférence inférieur à 20, ou un mélange desdits composés de formule (I).

17. Utilisation telle que définie à la revendication 16, pour laquelle, dans la formule (I), R₁ représente un radical choisi de préférence parmi les radicaux alkyles linéaires ou ramifiés comportant de 8 à 18 atomes de carbone.

18. Utilisation telle que définie à la revendication 17, pour laquelle, l'agent tensioactif, est un mélange d'alcools gras comportant 11, 13 et 15 atomes de carbone, éthoxylés et propoxylés, avec un indice d'OE environ compris entre 6 et 7 et un indice d'OP environ égal à 3.

19. Utilisation telle que définie à l'une des revendications 14 à 18, pour laquelle la solution aqueuse comprend comme composé générateur d'oxygène actif, de l'acide peracétique, du peroxyde d'hydrogène et de l'acide acétique.

20. Utilisation telle que définie à la revendication 19, pour laquelle dans la solution mise en oeuvre, l'acide peracétique est à une concentration comprise entre environ 0,0025% en poids et environ 0,05% en poids, le peroxyde d'hydrogène est à une concentration comprise entre environ 0,025% en poids et environ 0,5% en poids et l'acide acétique est à une concentration comprise entre environ 0,05% en poids et environ 0,5% en poids.

21. Utilisation telle que définie à la revendication 20, pour laquelle dans la solution mise en oeuvre, l'acide peracétique est à une concentration comprise entre environ 0,01% en poids et environ 0,025% en poids, le peroxyde d'hydrogène est à une concentration comprise entre environ 0,1% en poids et environ 0,25% en poids et l'acide acétique est à une concentration comprise entre environ 0,1% en poids et environ 0,25% en poids.

22. Utilisation telle que définie à l'une des revendications 16 à 21, pour laquelle la solution aqueuse mise en oeuvre comprend entre environ 0,002% en poids et environ 0,01% en poids, de préférence entre environ 0,003% en poids et 0,005% en poids d'un composé de formule(I) ou d'un mélange de composés de formule (I).

23. Utilisation d'une solution aqueuse comprenant de 0,1% à 1% en poids, d'acide peracétique, de 2% à 10% en poids de peroxyde d'hydrogène et de 2 à 10% en poids d'acide acétique et de 0,02% en poids à 0,5% en poids, d'un composé de formule (I) ou d'un mélange de composés de formule (I) telle que définie à l'une des revendications 17 à 22, pour, après une dilution au sein de l'appareil d'hémodialyse, comprise entre le 1/10ème et le 1/50ème et plus particulièrement comprise entre le 1/30ème et le 1/40ème, nettoyer, détartrer et désinfecter un, plusieurs ou tous les éléments de la chaîne de dialyse d'un appareil d'hémodialyse.

24. Utilisation telle que définie à la revendication 23, d'une solution aqueuse comprenant de 0,1% à 0,6% en poids, d'acide peracétique, de 3% à 7% en poids de peroxyde d'hydrogène et de 3% à 6% en poids d'acide acétique et de 0,05% en poids à 0,20% en poids, d'un agent tensioactif consistant essentiellement en un mélange d'alcools gras comportant 11, 13 et 15 atomes de carbone, éthoxylés et propoxylés, avec un indice d'OE environ compris entre 6 et 7 et un indice d'OP environ égal à 3.

25. Procédé tel que défini à l'une des revendications 7 à 13, comprenant en outre, une étape de rinçage postérieure à la séquence de traitement.

26. Procédé pour conserver la chaîne de dialyse à l'abri du développement du bio-film, caractérisé en ce que ladite chaîne est remplie avec une solution nettoyante et désinfectante telle que définie à l'une des revendications 14 à 23 et est laissée au repos sans recirculation de ladite solution.

27. Solution aqueuse comprenant de 0,1% à 1% en poids, d'acide peracétique, de 2% à 10% en poids de peroxyde d'hydrogène et de 2 à 10% en poids d'acide acétique et de 0,02% en poids à 0,5% en poids, d'un composé de formule (I) ou d'un mélange de composés de formule (I) telle que définie à l'une des revendications 16 à 18.

28. Solution aqueuse comprenant de 0,1% à 0,6% en poids, d'acide peracétique, de 3% à 7% en poids de peroxyde d'hydrogène et de 3% à 6% en poids d'acide acétique et de 0,05% en poids à 0,20% en poids, d'un agent tensioactif consistant essentiellement en un mélange d'alcools gras comportant 11, 13 et 15 atomes de carbone, éthoxylés et propoxylés, avec un indice d'OE environ compris entre 6 et 7 et un indice d'OP environ égal à 3.

29. Solution aqueuse telle que définie à l'une des revendications 27 ou 28, comprenant en outre un ou plusieurs additifs choisis parmi les agents stabilisants, les acides forts, les agents séquestrant, les inhibiteurs de corrosion ou les capteurs de radicaux libres.
